# EUROPEAN PATENT SPECIFICATION

(11) **EP 2 038 411 B1**
(45) Date of publication and mention of the grant of the patent: **26.02.2014**
(21) Application number: 07765926.6
(22) Date of filing: 19.06.2007
(51) Int. Cl.: C12N 9/86, A61K 38/46, C12N 1/21

(54) **MODIFIED BETA-LACTAMASE AND METHOD FOR ITS PREPARATION**
MODIFIZIERTE BETA-LACTAMASE UND VERFAHREN ZUR HERSTELLUNG DAVON
BETA-LACTAMASE MODIFIEE ET SON PROCEDE DE PREPARATION

(30) Priority: 21.06.2006 FI 20065431
(43) Date of publication of application: 25.03.2009
(73) Proprietor: Synthetic Biologics, Inc., Ann Arbor, MI 48104 (US)
(72) Inventor: KÄÄRIÄINEN, Susanna, FI-02140 Espoo (FI); WICKSTRAND, Nina, FI-00660 Helsinki (FI); KOSKI, Pertti, FI-00870 Helsinki (FI)
(74) Representative: Kolster Oy Ab
(86) International application number: PCT/FI2007/050372
(87) International publication number: WO 2007/147945

(56) References cited:
- WO-A1-03/040352
- WO-A2-2004/016248
- WALTHER-RASMUSSEN J ET AL: "Terminal truncations in Amp C beta-lactamase from a clinical isolate of Pseudomonas aeruginosa" EUROPEAN JOURNAL OF BIOCHEMISTRY, BLACKWELL PUBLISHING, BERLIN, DE, vol. 263, no. 2, 1 July 1999 (1999-07-01), pages 478-485, XP008101474 ISSN: 0014-2956
- SIMM A ET AL: "Characterization of Monomeric L1 Metallo-beta-lactamase and the Roke of the N-terminal Extension in Negative Cooperativity and Antibiotic Hydrolysis" JOURNAL OF BIOLOGICAL CHEMISTRY, AMERICAN SOCIETY FOR BIOCHEMISTRY AND MOLECULAR BIOLOGY, INC, US, vol. 277, no. 27, 5 July 2002 (2002-07-05) , pages 24744-24752, XP008101477 ISSN: 0021-9258 [retrieved on 2002-04-08]
- GARAU G ET AL: "Update of the Standard Numbering Scheme for Class B beta-Lactamases" ANTIMICROBIAL AGENTS AND CHEMOTHERAPY, AMERICAN SOCIETY FOR MICROBIOLOGY, WASHINGTON, DC, US, vol. 48, no. 7, 1 July 2004 (2004-07-01), pages 2347-2349, XP008101475 ISSN: 0066-4804
- STIEFEL U ET AL: "Orally Administered Recombinant Metallo-beta-Lactamase Preserves Colonization Resistance of Piperacillin-Tazobactam-Treated Mice" ANTIMICROBIAL AGENTS AND CHEMOTHERAPY, AMERICAN SOCIETY FOR MICROBIOLOGY, WASHINGTON, DC, US, vol. 49, no. 12, 1 December 2005 (2005-12-01), pages 5190-5191, XP008101476 ISSN: 0066-4804
- HARMOINEN J ET AL: "Orally administered targeted recombinant beta-lactamase prevents ampicillin-induced selective pressure on the gut microbiota: A novel approach to reducing antimicrobial resistance" ANTIMICROBIAL AGENTS AND CHEMOTHERAPY,, vol. 48, no. 1, 1 January 2004 (2004-01-01), pages 75-79, XP008109230
- CRAWFORD P A ET AL: "Over-expression, purification, and characterization of metallo-beta-lactamase ImiS from Aeromonas veronii bv. sobria" PROTEIN EXPRESSION AND PURIFICATION, ACADEMIC PRESS, SAN DIEGO, CA, vol. 36, no. 2, 1 August 2004 (2004-08-01) , pages 272-279, XP004520292 ISSN: 1046-5928
- STIEFEL U. ET AL.: 'Orally Administered Recombinant Metallo-beta-Lactamase Preserves Colonization Resistance of Piperacillin-Tazobactam-Treated Mice' ANTIMICROBIAL AGENTS AND CHEMOTHERAPY vol. 49, no. 12, 2005, pages 5190 - 5191, XP008101476
- SIMM A. ET AL.: 'Characterization of Monomeric L1 Metallo-beta-lactamase and the Roke of the N-terminal Extension in Negative Cooperativity and Antibiotic Hydrolysis' THE JOURNAL OF BIOLOGICAL CHEMISTRY vol. 277, no. 27, 2002, pages 24744 - 24752, XP008101477
- GARAU G. ET AL.: 'Update of the Standard Numbering Scheme for Class B beta-Lactamases' ANTIMICROBIAL AGENTS AND CHEMOTHERAPY vol. 48, no. 7, 2004, pages 2347 - 2349, XP008101475
- WALTHER-RASMUSSEN J. ET AL.: 'Terminal truncations in Amp C beta-lactamase from a clinical isolate of Pseudomonas aeruginosa' EUROPEAN JOURNAL OF BIOCHEMISTRY vol. 263, no. 2, 1999, pages 478 - 485, XP008101474
- CARFI A. ET AL.: 'The 3-D structure of a zinc metallo-beta-lactamase from Bacillus cereus reveals a new type of protein fold' THE EMBO JOURNAL vol. 14, no. 20, 1995, pages 4914 - 4921, XP008101464

### SEQUENCE LISTING

<110> IPSAT Therapies
<120> Modified beta-lactamase and method for its preparation
<130> 2061011
<160> 3
<170> Patent In version 3.2
<210> 1
   <211> 257
   <212> PRT
   <213> Bacillus cereus
<220>
   <221> mat_peptide
   <222> (31)..(257)
<400> 1
   Met Lys Lys Asn Thr Leu Leu Lys Leu Gly Val Cys Val Ser Leu Leu
   -30                 -25                 -20                 -15
   Gly Ile Thr Gln Phe Val Ser Thr Ile Ser Ser Val Lys Ala Glu Gln
                   -10                 -5                  -1  1
   Lys Leu Glu Gln Ile Val Ile Lys Asn Glu Thr Gly Thr Ile Ser Ile
           5                   10                  15
   Ser Gln Leu Asn Lys Asn Val Trp Val His Thr Glu Leu Gly Tyr Phe
       20                  25                  30
   Asn Gly Glu Ala Val Pro Ser Asn Gly Leu Val Leu Asn Thr Ser Lys
   35                  40                  45                  50
   Gly Leu Val Leu Val Asp Ser Ser Trp Asp Asn Lys Leu Thr Lys Glu
                   55                  60                  65
   Leu Ile Glu Met Val Glu Lys Lys Phe Gln Lys Arg Val Thr Asp Val
               70                  75                  80
   Ile Ile Thr His Ala His Ala Asp Arg Ile Gly Gly Ile Thr Ala Leu
           85                  90                  95
   Lys Glu Arg Gly Ile Lys Ala His Ser Thr Ala Leu Thr Ala Glu Leu
       100                 105                 110
   Ala Lys Asn Ser Gly Tyr Glu Glu Pro Leu Gly Asp Leu Gln Thr Ile
   115                 120                 125                 130
   Thr Ser Leu Lys Phe Gly Asn Thr Lys Val Glu Thr Phe Tyr Pro Gly
                   135                 140                 145
   Lys Gly His Thr Glu Asp Asn Ile Val Val Trp Leu Pro Gln Tyr Gln
   150 155 160
   Ile Leu Ala Gly Gly Cys Leu Val Lys Ser Ala Glu Ala Lys Asp Leu
   165 170 175
   Gly Asn Val Ala Asp Ala Tyr Val Asn Glu Trp Ser Thr Ser Ile Glu
   180 185 190
   Asn Val Leu Lys Arg Tyr Gly Asn Ile Asn Ser Val Val Pro Gly His
   195 200 205 210
   Gly Glu Val Gly Asp Lys Gly Leu Leu Leu His Thr Leu Asp Leu Leu
   215 220 225
   Lys
<210> 2
   <211> 759
   <212> DNA
   <213> Bacillus cereus
<220>
   <221> sig_peptide
   <222> (1) .. (93)
<220>
   <221> CDS
   <222> (1) .. (756)
<220>
   <221> mat_peptide
   <222> (94)..(756)
<400> 2
   atg att caa aaa cga aag cgg aca gtt tcg ttc aga ctt gtg ctt atg      48
   Met Ile Gln Lys Arg Lys Arg Thr Val Ser Phe Arg Leu Val Leu Met
       -30                 -25                 -20
   tgc acg ctg tta ttt gtc agt ttg ccg att aca aaa aca tca gcg caa      96
   Cys Thr Leu Leu Phe Val Ser Leu Pro Ile Thr Lys Thr Ser Ala Gln
   -15                 -10                 -5                  -1   1
   gct tcc aaa aca gag acg gga acc att tca ata tct cag tta aac aag     144
   Ala Ser Lys Thr Glu Thr Gly Thr Ile Ser Ile Ser Gln Leu Asn Lys
               5                   10                  15
   aat gta tgg gtt cat acg gag tta ggt tat ttt aat gga gaa gca gtt     192
   Asn Val Trp Val His Thr Glu Leu Gly Tyr Phe Asn Gly Glu Ala Val
           20                  25                  30
   cct tcg aac ggt cta gtt ctt aat act tct aaa ggg cta gta ctt gtt     240
   Pro Ser Asn Gly Leu Val Leu Asn Thr Ser Lys Gly Leu Val Leu Val
       35                  40                  45
   gat tct tct tgg gat aac aaa tta acg aag gaa cta ata gaa atg gta     288
   Asp Ser Ser Trp Asp Asn Lys Leu Thr Lys Glu Leu Ile Glu Met Val
   50                  55                  60                  65
   gaa aag aaa ttt cag aag cgc gta acg gat gtc att att aca cat gcg     336
   Glu Lys Lys Phe Gln Lys Arg Val Thr Asp Val Ile Ile Thr His Ala
                   70                  75                  80
   cac gct gat cga att ggc gga ata aca gcg ttg aaa gaa aga ggc att     384
   His Ala Asp Arg Ile Gly Gly Ile Thr Ala Leu Lys Glu Arg Gly Ile
               85                  90                  95
   aaa gcg cat agt aca gca tta acc gca gaa cta gca aag aac agt gga     432
   Lys Ala His Ser Thr Ala Leu Thr Ala Glu Leu Ala Lys Asn Ser Gly
           100                 105                 110
   tat gaa gag ccg ctt gga gat tta caa aca att acg agt tta aag ttt     480
   Tyr Glu Glu Pro Leu Gly Asp Leu Gln Thr Ile Thr Ser Leu Lys Phe
       115                 120                 125
   ggc aat aca aaa gta gaa acg ttc tat cca ggg aaa gga cat aca gaa     528
   Gly Asn Thr Lys Val Glu Thr Phe Tyr Pro Gly Lys Gly His Thr Glu
   130                 135                 140                 145
   gat aat att gtt gtt tgg ttg cca caa tat caa att tta gct gga ggc     576
   Asp Asn Ile Val Val Trp Leu Pro Gln Tyr Gln Ile Leu Ala Gly Gly
                   150                 155                 160
   tgt tta gta aaa tct gcg gaa gct aaa gat tta gga aat gtt gcg gat     624
   Cys Leu Val Lys Ser Ala Glu Ala Lys Asp Leu Gly Asn Val Ala Asp
               165                 170                 175
   gcg tat gta aat gaa tgg tct aca tcg att gag aat gtg ctg aag cga     672
   Ala Tyr Val Asn Glu Trp Ser Thr Ser Ile Glu Asn Val Leu Lys Arg
           180                 185                 190
   tat gga aat ata aat tcg gta gta cct ggt cat gga gaa gta gga gac     720
   Tyr Gly Asn Ile Asn Ser Val Val Pro Gly His Gly Glu Val Gly Asp
       195                 200                 205
   aag gga tta ctt tta cat aca ttg gat tta tta aaa taa                 759
   Lys Gly Leu Leu Leu His Thr Leu Asp Leu Leu Lys
   210                 215                 220
<210> 3
   <211> 252
   <212> PRT
   <213> Bacillus cereus
<400> 3
   Met Ile Gln Lys Arg Lys Arg Thr Val Ser Phe Arg Leu Val Leu Met
       -30                 -25                 -20
   Cys Thr Leu Leu Phe Val Ser Leu Pro Ile Thr Lys Thr Ser Ala Gln
   -15                 -10                 -5              -1  1
   Ala Ser Lys Thr Glu Thr Gly Thr Ile Ser Ile Ser Gln Leu Asn Lys
               5                   10                  15
   Asn Val Trp Val His Thr Glu Leu Gly Tyr Phe Asn Gly Glu Ala Val
           20                  25                  30
   Pro Ser Asn Gly Leu Val Leu Asn Thr Ser Lys Gly Leu Val Leu Val
       35                  40                  45
   Asp Ser Ser Trp Asp Asn Lys Leu Thr Lys Glu Leu Ile Glu Met Val
   50                  55                  60                  65
   Glu Lys Lys Phe Gln Lys Arg Val Thr Asp Val Ile Ile Thr His Ala
                   70                  75                  80
   His Ala Asp Arg Ile Gly Gly Ile Thr Ala Leu Lys Glu Arg Gly Ile
               85                  90                  95
   Lys Ala His Ser Thr Ala Leu Thr Ala Glu Leu Ala Lys Asn Ser Gly
           100                 105                 110
   Tyr Glu Glu Pro Leu Gly Asp Leu Gln Thr Ile Thr Ser Leu Lys Phe
       115                 120                 125
   Gly Asn Thr Lys Val Glu Thr Phe Tyr Pro Gly Lys Gly His Thr Glu
   130                 135                 140                 145
   Asp Asn Ile Val Val Trp Leu Pro Gln Tyr Gln Ile Leu Ala Gly Gly
                   150                 155                 160
   Cys Leu Val Lys Ser Ala Glu Ala Lys Asp Leu Gly Asn Val Ala Asp
               165                 170                 175
   Ala Tyr Val Asn Glu Trp Ser Thr Ser Ile Glu Asn Val Leu Lys Arg
           180                 185                 190
   Tyr Gly Asn Ile Asn Ser Val Val Pro Gly His Gly Glu Val Gly Asp
       195                 200                 205
   Lys Gly Leu Leu Leu His Thr Leu Asp Leu Leu Lys
   210                 215                 220

## Description

### Field of the Invention

Various antibiotics are used in the treatment of bacterial infections. However, the antibiotics do not only attack pathogens, but they also affect the normal bacterial flora, leading to adverse side effects e.g. in the patient's intestine. These side effects can be reduced by administering enzymes capable of degrading residual antibiotic in the intestine. The present invention relates to modified metallo-beta-lactamases that are useful in treating and preventing adverse effects of antibiotics having a beta-lactam ring, or in the preparation of such enzymes. The invention is also directed to a method for preparing the modified beta-lactamases as well as to nucleotide molecules, vectors and host cells useful therein.

### Background of the Invention

Beta-lactamase enzymes represent a major mechanism of resistance among bacteria to beta-lactam antibiotics, which include penicillins, cephalosporins and carbapenems. These enzymes catalyse the irreversible hydrolysis of the amide bond of the beta-lactam ring to create ineffective antimicrobial agents. On the basis of molecular structure classification and catalytic mechanisms the beta-lactamases can be divided into four classes: A, B, C, and D. Classes A, C and D are serine enzymes and comprise the majority of the beta-lactamases (Ambler, 1980). These enzymes generally inactivate penicillins or cephalosporins and often show a preference for one of these two antibiotics.

Class B beta-lactamases are metallo-enzymes that require one or two zinc ions as a cofactor for enzyme activity. The metallo-beta-lactamases constitute group 3 in the Bush-Jacoby-Madeiros functional classification (Bush, 1998). This schema is primarily based on substrate profiles, their sensitivity to EDTA and their resistance to serine beta-lactamase inhibitors. Based on structural similarities in the region that coordinates zinc binding, metallo-beta-lactamases can be divided into three subgroups, B1, B2, and B3 (Galleni et al., 2001). Subgroup B1 possesses three histidines and one cysteine as the key zinc coordinating residues. Crystallographic structures have been described for many subgroup B1 enzymes such as Bcll of *Bacillus cereus* (Carfi et al, 1995 and 1998a), CcrA of *Bacteroides fragilis* and (Carfi et al., 1998b) and IMP-1 of *Pseudomonas aeruginosa* (Concha *et al.,* 2000). Correspondingly, subgroup B2 lactamases have an arginine residue, instead of histidine, at the first position of the principal zinc binding motif, NXHXD. Recently, the first crystal structure of a subgroup B2 enzyme (CphA) has been solved by Garau *et al.* (2005). Subgroup B3 contains enzymes with multimeric structure (Walsh *et al.,* 2005).

Metallo-beta-lactamases show a broad spectrum substrate profile including penicillins and cephalosporins, and they are resistant to the action of common conventional serine beta-lactamase inhibitors such as clavulanic acid sulbactam and tazobactam. Furthermore, unlike most of the serine betalactamases, metallo-beta-lactamases have the capability to hydrolyze carbapenems such as meropenem and imipenem. Various numbers of bacteria are known to produce metallo-beta-lactamases. They are commonly expressed among the *Enterobacteriae* genus (including *Serratia marcescens, Klebsiella pneumoniae, Citrobacter freudii, Shigella flexnen*), *Pseudomonas aeruginosa, Stenobacterium maltophila, Acinetobacter* genus, *Bacteroides fragilis, Bacillus cereus, Flavobacteruim odoratum,* and *Bacteroides fragilis* (Walsh *et a*l., 2005).

Beta-lactamases can be utilized as pharmaceutical proteins to inactivate unabsorbed beta-lactams in the gastro intestinal tract in order to prevent the beta-lactam induced adverse effects including alterations in intestinal normal microbiota and the overgrowth of beta-lactam resistant bacteria (WO93/13795, WO2004/016248). For efficient beta-lactamase therapy in the small intestinal tract the enzyme should be resistant to the action of intestinal proteases in the presence of bile acids and preserve high enzymatic activity at a wide range of pH (5.5-7.5).

The feasibility of targeted enzyme therapy in canine and mouse models was demonstrated by employing a *Bacillus licheniformis* serine betalactamase during parenteral ampicillin medication (Harmoinen *et al.,* 2004, Mentula *et al.,* 2004, Stiefel *et al.,* 2003). However, the substrate profile of this enzyme essentially limits its use as a drug substance since it has poor capacity to hydrolyze cephalosporins, carbapenems or penicillins in the presence of beta-lactamase inhibitors. Consequently, a new protease resistant betalactamase enzyme with broad beta-lactam spectrum is indispensable to extend the use of beta-lactamase therapy among hospitalized patients under intravenous medication with various beta-lactams.

Metallo-beta-lactamases are known to inactivate various types of beta-lactams and they are resistant to inhibitors of serine beta-lactamases. *Bacillus cereus* strains are known to produce metallo-beta-lactamase that belongs to group B1. A semi purified recombinantly produced metallo-betalactamase sample of a clinical *Bacillus cereus* 98ME1552 isolate was shown to eliminate the overgrowth of potential pathogenic bacteria in a mouse model (Stiefel *et al.,* 2005). However, the present inventors found that this metallo-beta-lactamase preparation contained a mixture of beta-lactamase variants, which declines its value as a pharmaceutical protein, since variations of a drug substance reduce the robustness of the production process, Increase batch to batch variations, and make clinical trials difficult, which of course has a negative impact on its registration as a medicament.

Heterogeneity has previously been found also among betalactamases from *Pseudomonas aeruginosa* (Walther-Rasmussen et al., 1999), whereas no heterogeneity of the N-terminal end was found in expressing metallo-beta-lactamase from *Aeromonas veronii* bv. *sobria* in *E. coli* (Crawford et al., 2004). The role of an amino terminal extension in a metallo-betalactamase from *Stenotrophomonas maltophilia* is disclosed in Simm et al., 2002. The beta-lactamases in these references differ from that of the present invention.

The present invention now provides means for reducing the amino terminal heterogenecity that was found to be associated with recombinant production of a metallo-beta-lactamase. The invention further provides modified metallo-beta-lactamases that can be produced in substantially pure form and that can be used in the manufacture of pharmaceutical compositions.

### Summary of the Invention

The invention provides a modified metallo-beta-lactamase protein having the general formula:

NH₂-K-T-E-ΔBL-COOH (I)

wherein
K is lysine
T is threonine
E is glutamic acid, and
ΔBL is a metallo-beta-lactamase protein that has been truncated at the amino terminal end so as to leave four beta-strands before the first alpha-helix of the predicted secondary structure of said protein, and the sequence E - ΔBL has at least 70 % identity to the amino acid sequence of sequential amino acid residues 6-221 of SEQ ID NO:3.

The invention further provides an isolated nucleotide molecule comprising a nucleotide sequence encoding said modified metallo-beta-lactamase protein, as well as an expression vector containing the nucleotide molecule, and a host cell capable of expressing the metallo-beta-lactamase protein encoded by the nucleotide molecule.

The invention also provides a modified metallo-beta-lactamase protein having the general formula

NH₂-E-ΔBL-COOH (II)

wherein
E and ABL are as defined above.

The invention still further provides a method of preparing a modified metallo-beta-lactamase protein, said method comprising culturing said host cell under conditions enabling the expression of a metallo-beta-lactamase having the general formula:

NH₂-K-T-E-ΔBL-COOH, (I)

wherein
K is lysine
T is threonine
E is glutamic acid, and
ΔBL is a metallo-beta-lactamase protein that has been truncated at the amino terminal end so as to leave four beta-strands before the first alpha-helix of the predicted secondary structure of said protein, and the sequence E - ΔBL has at least 70 % identity to the amino acid sequence of sequential amino acid residues 6-221 of SEQ ID NO:3.
and conducting post translational modification resulting in a modified metallo-beta-lactamase having the general formula:

   NH₂-E-ΔBL-COOH, (II)
wherein E and ΔBL are as defined above, and
optionally isolating and purifying the post translationally modified protein obtained.

As additional aspects the invention provides a pharmaceutical composition comprising the modified metallo-beta-lactamase protein of formula II, the modified metallo-beta-lactamase of formula II for use as a medicament, and the use of the modified metallo-beta-lactamase of formula II for the manufacture of a medicament for elimination of beta-lactam antibiotic induced adverse effects in the intestinal tract.

Finally a method of treating beta-lactam antibiotic induced adverse effects in the intestinal tract comprising administering an effective amount of the modified metallo-beta-lactamase of formula II, or the pharmaceutical composition containing it, to a person in need thereof is described. Specific embodiments of the invention are set forth in the dependent claims.

Other objects, details and advantages of the present invention will become apparent from the following drawings, detailed description and examples.

### Brief Description of the Drawings

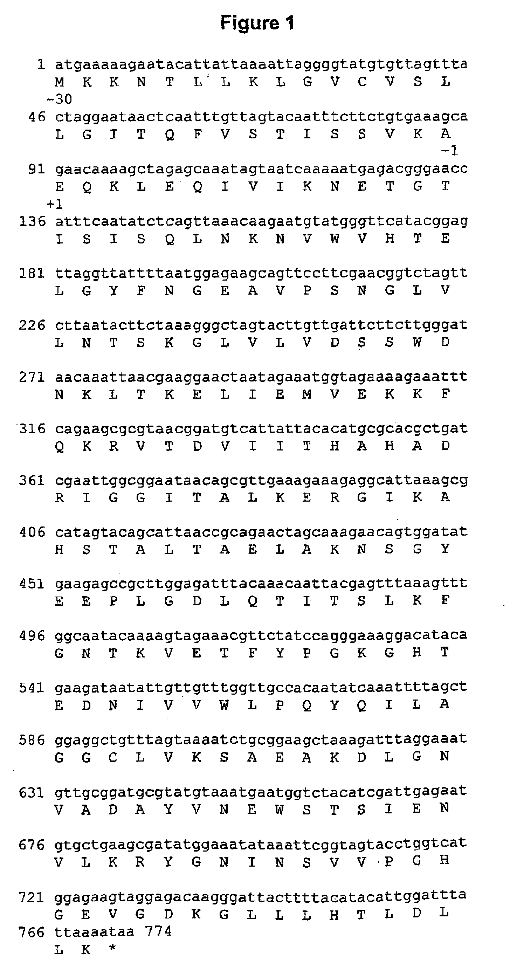

Figure 1 shows the complete nucleotide sequence and the deduced amino acid sequence of the *B. cereus* 98ME1552 beta-lactamase gene.

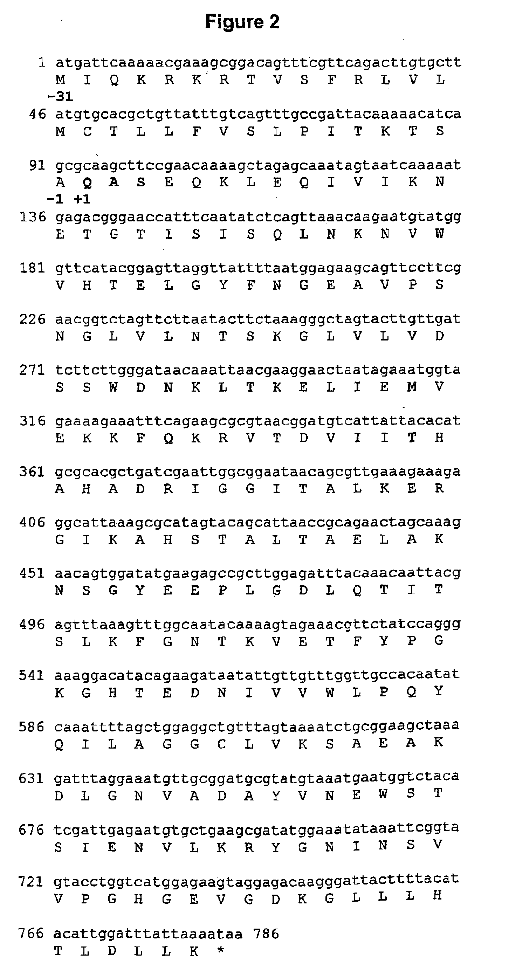

Figure 2 shows the complete nucleotide sequence and the deduced amino acid sequence of the *B. cereus* 98ME1552 beta-lactamase gene derived from the pRSH315 expression construct. The cleavage site of the 31 amino acid residues long signal sequence of *Bacillus amyloliquefaciens* is predicted to occur between alanine at position -1 and glutamine at position +1. The NH₂-terminal extension of a NH₂-QAS-tripeptide that is derived from the *Hind* III cloning site is expressed in bold.

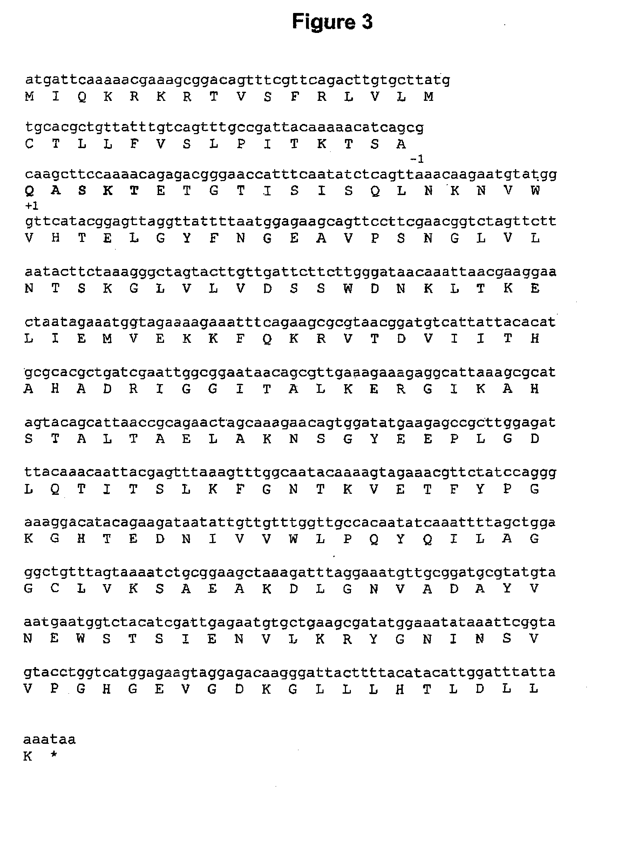

Figure 3 shows the complete nucleotide sequence and the deduced amino acid sequence of the *B. cereus* 98ME1552 beta-lactamase gene derived from the pRSH318 expression construct. The cleavage of a 31 amino acid residues long signal sequence of *Bacillus amylofiquefaciens* is predicted to occur between alanine at position -1 and glutamine at position +1. The NH₂-terminal extension of the NH₂-QAS-tripeptide derived from the *Hind* III cloning site and the KT insertion are shown in bold.

### Detailed Description of the Invention

A metallo-beta-lactamase preparate was first produced in a *Bacillus subtilis* production system containing an expression construct of the complete metallo-beta-lactamase gene encoding the complete metallo-beta enzyme. Detailed mass spectrometry analysis revealed that the metallo enzyme preparate included various numbers of enzyme variants with heterogeneous amino terminal sequences. The variations at the amino terminus sequences were believed to be a result of post translational modification of host cell proteases. The observed alterations in the amino acid sequence of the metallo enzyme increase batch to batch variations of enzyme that has been produced in a *Bacillus subtilis* production system and thus in regulatory perspective reduce its use as a pharmaceutical protein. Therefore molecular biological means for reducing the observed variations in the amino terminal sequence of the metallo-beta-lactamase enzyme were studied.

The amino terminal region is expected to have no essential influence on the catalytic properties of the enzyme. In addition the amino terminal region was found to be exposed to post translational modifications of proteases in the *Bacillus subtilis* production system in which the recombinant protein is secreted outside the bacterial cell. In order to reduce this microheterogeneity in the amino terminal region, the nucleotide sequence encoding this predicted region was deleted by a PCR method. However, mere deletion by itself did not lead to a significant reduction of the amino terminal heterogeneity. Surprisingly, however, the deletion combined with insertion of a dipeptide, which was designed to assist post translational modification, led to a single metallo-betalactamase variant produced with an equally modified amino terminus.

This invention relates in general to a modified beta-lactamase useful as a pharmaceutical protein, and also to a recombinant beta-lactamase intermediate that is produced by truncation and insertion of a dipeptide at its amino terminal resulting in reduced numbers of beta-lactamase variants. These modifications facilitate the recombinant production of the enzyme in homologous form for use as a drug substance in beta-lactamase therapy for elimination of beta-lactams (cephalosporins, carbapenems, and penicillins in the presence or absence of known beta-lactamase inhibitors such as clavulanic acid, sulbactam and tazobactam) induced side effects. In particular the invention relates to targeted post translational modification of active metallo-beta-lactamase by truncation and insertion of a dipeptide at the amino terminal region to reduce amino terminal heterogeneity in a *Bacillus subtilis* production system.

"Metallo-beta-lactamases" as used herein refer to class B betalactamases i.e. beta-lactamases that require at least one bivalent metal ion (Zn²⁺) for activity; and constitute group 3 in the in the Bush-Jacoby-Madeiros functional classification (Bush, 1998). Based on the structural similarities in the region that coordinates zinc binding, metallo-beta-lactamases can be divided into three subgroups, B1, B2, and B3 (Galleni et al., 2001). Preferably the metallo-beta-lactamase of the invention belongs to subgroup B1. This subgroup possesses the key zinc coordinating residues of three histidines and one cysteine. More details about the subgroups are set forth in the background part of this specification.

The metallo-beta-lactamases are members of a large, diverse, superfamily of proteins that share a similar four layered αβ/βα structure. The polypeptide chain is divided into two domains, which comprise strands and helices in the following order: β₁ β₂ β₃ β₄ β₅ α₁ β₆ α₂ β₇ α₃ and β₈ β₉ β₁₀ β₁₁ α₄ β₁₂ α₅, respectively (Carfi *et al.,* 1995 and 1998a, and Galleni *et al.,* 2001). The metallo-beta-lactamases of the invention are truncated at the amino terminal end prior to the second beta strand. Preferably they are truncated between the first and second beta strands, and in particular immediately in front of the amino acid E between the first and second beta strands according to the crystallographic structure above.

Garau *et al.,* 2004 have set forth a standard numbering scheme for class B beta-lactamases (BBL) by analogy to Ambler beta-lactamase number-ing (ABL) for serine beta-lactamases (Ambler, 1980). The BBL numbering scheme is derived from a structural alignment of known metallo-betalactamase X-ray structures in order to discover conserved regions among various metallo beta-lactamase groups having low degree of identity in primary amino acid sequence level. The study of Garau *et al.* revealed that the first conserved fragment forms the second beta sheet structure in *Bacillus cereus* BcII metallo beta-lactamase set forth by Carfi *et al.* 1995, and Carfi et al., 1998a. Consequently, the β₁-sheet of *B. cereus* metallo beta-lactamase appears to be non-essential for enzyme function, because it is not be present in all metallo beta-lactamase groups. However, the amino terminal region of all metallo beta-lactamases possess a secondary structure of four conserved beta sheets forming fragments before the first alpha helix forming fragment. Thus the truncation site for the metallo beta-lactamase may be defined as one leaving four beta strands before the first alpha helix of the predicted secondary structure regardless of the original presence of β₁ or not.

In the present context conventional one-letter amino acid codes and are used. Thus, A denotes alanine, R denotes arginine, N denotes asparagine, D denotes aspartic acid, C denotes cysteine, E denotes glutamic acid, Q denotes glutamine, G denotes glycine, H denotes histidine, I denotes isoleucine, L denotes leucine, K denotes lysine, M denotes methionine, F denotes phenylalanine, P denotes proline, S denotes serine, T denotes threonine, W denotes tryptophan, Y denotes tyrosine, and V denotes valine. The amino acid sequences are shown with the amino terminus to the left and carboxyl terminus to the right. NH₂- and -COOH may or may not be indicated.

According to one embodiment of the invention a host cell is transformed with an expression vector capable of expressing a beta-lactamase having the general formula I:

NH₂-K-T-E-ΔBL-COOH, (I)

wherein K is lysine, T is threonine, E is glutamic acid, and ΔBL is a metallobeta-lactamase protein that has been truncated at the amino terminal end prior to the second beta-strand of said protein, whereby a protein having the general formula II:

NH₂-E-ΔBL-COOH, (II)

is formed as a result of post translational modification of the protein of formula I. The protein of the general formula I is conveniently produced by ligating a KT encoding DNA sequence to an E - ΔBL encoding DNA sequence, wherein E-ΔBL is a *Bacillus* and in particular a *B. cereus* metallo-beta-lactamase that has been truncated at the amino terminal end prior to the second beta strand immediately in front of a glutamic acid residue E. In particular the nucleotide construct comprises the sequential nucleotides 94 - 756 of SEQ ID NO: 2.

The length of the amino terminal region without rigid structure varies between metallo-beta-lactamase groups and within each subclass. The metallo-beta-lactamases of *Bacillus* spp also possess variations in the amino terminal sequence prior to the second beta strand, but most enzymes have a conserved glutamic acid (E) at position +12 (see Table 1). Consequently, the deletion coupled with the KT insertion adjacent to E can be applied to known *Bacillus* metallo-beta-lactamases, in which case the first eleven amino terminal amino acids are deleted from the N-terminal end of the naturally occurring mature beta-lactamase protein. According to one specific embodiment of the invention the beta-lactamase is truncated between the amino acid KN and E, and especially between VIKN and E, or VMKN and E.

**Table 1. Comparison of the amino terminal sequences between Bacillus sp metallo-beta-lactamases and P2A.**

| **Protein** | ***Bacillus* strain** | **Amino terminal region** * |
|---|---|---|
| P2A | *B. cereus* 98ME1552 | |
| Q2AJV1 | *B. weihenstephanensis* KBAB4 | |
| P10425 | *Bacillus sp.* (strain 170) | |
| Q734F3 | *B*. *cereus* ATCC 10987 | |
| P04190 | *B. cereus* 569/H | |
| Q81AW2 | *B. cereus* ATCC 14579 / DSM 31 | |
| Q93T40 | *B. anthracis* Sterne | |
| Q6SPY5 | Penicillin resistant *B. anthracis* | |
| Q4MXZ5 | *B. cereus* G9241 | |
| P14488 | *B. cereus* 5/B/6 | |
| Q6HFY5 | *B. thuringiensis* 97-27 | |

| | | |
|---|---|---|
| * The amino acid substitutions are bolded and the conserved glutamic acid (E) is shaded. The first beta strand for metallo-beta-lactamases is once underlined, and the second beta strand is twice underlined. | | |

According to a specific embodiment of the invention E - ΔBL has at least 80, 90, 95, 98 or 99 % identity to the amino acid sequence of sequential amino acid residues 6-221 of SEQ ID NO: 3. Sequence identity may be determined using BLAST (Basic Local Alignment Search Tools) as described in Altschul *et al .,* 1997. In particular E - ΔBL is a truncated form of a metallo-beta-lactamase having the sequence as set forth as SEQ ID NO: 1, or a beta-lactamase active variant or fragment thereof. It may e.g. be a truncated form of *B. cereus* metallo-beta-lactamase BcII. Preferably E - ΔBL has an amino terminal end that corresponds to amino acid residues 12-15, 12-19, or 12-23 of SEQ ID NO: 1, with the addition that the amino acid at position + 13 may be either T (threonine) or A (alanine).

By an amino acid sequence that is a "variant" of a specific amino acid sequence is meant an amino acid sequence that is not identical to the specific amino acid sequence, but contains at least some amino acid changes i.e. deletions, substitutions, inversions, insertions etc. that do not essentially affect the biological activity of the protein as compared to that of the specific amino acid sequence. Biological activity in this context refers to betalactamase activity. A variant may be a polypeptide that occurs naturally e.g. as an allelic variant within the same strain, species or genus, or it may have been generated by mutagenesis.

A "fragment" is understood to be part of a specific amino acid sequence that is long enough to have the desired biological activity i.e. betalactamase activity. In other words the fragment may be e.g. a subsequence of the specifically disclosed beta-lactamases.

The nucleotide construct encoding NH₂ - K - T - E - ΔBL-COOH may be inserted in an expression vector, and transformed into a host cell. The host cell is then cultivated under conditions enabling expression and post translational modification of the protein into NH₂- E - ΔBL-COOH, which thus may be obtained in substantially pure form, which means that at least 90 %, and preferably at least 95 %, in particutar at least 99 % of the metallo-betalactamase is present in a single form as NH₂- E - ΔBL-COOH. The post translational modification of the expressed protein, i.e. the cleavage of the dipeptide KT is catalyzed by proteases of the host. The host may be eukaryotic or prokaryotic, such as a bacterial, yeast or fungus cell. The bacterial host may be e.g *Escherichia coil.* Preferably the host belongs to *Bacillus* spp, and in particular it is *B. licheniformis* or *B. subtilis.* According to one preferred embodiment the NH₂ - K - T - E - ABL-COOH is expressed as a protein comprising a signal peptide, whereby the protein is secreted into the culture medium, and the signal peptide is first cleaved, and then the dipeptide. Minor other changes such as deamidation, oxidation, disulfide bond breakage or formation, isomerization, succinimidation, non-disulfide crosslinking, Maillard reaction, deglycosylation may occur in the protein during the production process or storage, and are acceptable as long as the beta-lactamase activity is not significantly affected.

The modified beta-lactamase NH₂ - E - ΔBL-COOH may originate from any bacterium capable of producing a metalio-beta-lactamase. Such bacteria are e.g. bacteria of the *Enterobacteriae* genus (*Serratia marcescens, Klebsiella pneumoniae, Citrobacter freudii, Shigella flexneri), Pseudomonas aeruginosa, Stenobacferium maltophila, Acinetobacter* genus, *Bacteroides fragllis, Bacillus cereus, Flavobacteruim odoratum,* and *Bacteroides fragilis.* Other possible sources are *Aeromonas, Legionella* and *Stenotrophomonas* species. The enzyme is truncated at the amino terminal end of the mature enzyme protein prior to the second beta strand at a position resulting in E as the N-terminal amino acid. If there is no appropriate residue E in the bacterial betalactamase, only the ABL part may be obtained from the bacterium, whereas a tripeptide KTE is coupled In front of ΔBL to from the protein NH₂ - K - T - E - ΔBL-COOH.

Preferably the modified beta-lactamase is derived from *Bacillus,* and especially from *B. cereus.* In particular it is a *B. cereus* beta-lactamase from which the first eleven N-terminal amino acids of the non-modified mature betalactamase protein have been deleted.

The modified beta-lactamase of the invention may be mixed with any pharmaceutically acceptable excipients or carriers to form a pharmaceutical composition. An amount of the modified beta-lactamase effective of eliminating the adverse effect of beta-lactam antibiotics in the intestine is then administered orally to a person being treated with one or more beta-lactam antibiotics. The beta-lactamase preparation may be administered before, simultaneously with, or after the antibiotic treatment. It is insensitive to serine betalactamase inhibitors, and may therefore be used to eliminate the antibiotic both in the presence and absence of such inhibitors.

The invention is illustrated by the following non-limiting examples. It should be understood, however, that the embodiments given in the description above and In the examples are for illustrative purposes only, and that various changes and modifications are possible within the scope of the claims.

### Example 1. Materials and methods

### Bacterial strains and growth conditions

The bacterial strains and their relevant genotypes and phenotypes are presented in Table 2.

**Table 2. Bacterial strains and their relevant genotypes and phenotypes**

| **Strain** | **Relevant genotype** | **Relevant phenotype** |
|---|---|---|
| *Bacillus subtilis* RS303 | *irpC2, sigG::cat* (Δ *sigG*) | Tryptophan auxotroph, asporogenic |
| *Bacillus subtilis* IH 6140 | *sacA321* | Reduced exoprotease level |
| *Bacillus subtilis* RS314 | *trpC2, sigG::cat,* (Δ *sigG*) | Tryptophan auxotroph, asporogenic, secretes metallo-beta-lactamase |
| | pRSH314 expression construct encoding the truncated metallo-beta-lactamase | |
| *Bacillus subtilis* RS315 | *trpC2, sigG::cat,* (Δ *sigG*) | Tryptophan auxotroph, asporogenic, secretes metallo-beta-lactamase |
| | pRSH315expression construct encoding the complete metallo-beta-lactamase gene | |
| *Bacillus subtilis* RS317 | *trpC2, sigG::cat,* (A *sigG*) | Tryptophan auxotroph, asporogenic, secretes metallo-beta-lactamase |
| | pRSH317expression construct encoding truncated metallobeta-lactamase | |
| *Bacillus subtilis* RS318 | *trpC2, sigG::cat,* (Δ *sigG*) | Tryptophan auxotroph, asporogenic, secretes metallo-beta-lactamase |
| | pRSH318 expression construct encoding truncated metallo-beta-lactamase | |
| *Bacillus cereus* 98ME1552 | | Multiresistant to various beta-lactams |

Bacteria were generally cultivated in complex Luria medium (5 grams of yeast extract, 10 grams of tryptone and 10 grams of sodium chloride per litre) supplemented with appropriate antibiotics as selection agents in shaking flask mode at +37°C. A synthetic culture medium supplemented with appropriate antibiotic was used in fermentations. A modified synthetic medium was used to generate competent *Bacillus subtilis* cells. Detailed composition of these media is described in WO03/040352.

### DNA techniques

Conventional DNA techniques including e.g. restrictions, agarose gel electrophoresis and ligations, were performed according to Sambrook and Russell (2001). The chromosomal DNA was isolated by the Marmur method (Marmur, 1961). The plasmid DNA was isolated by Qiagen plasmid Midi Kit according to manufacturer's instructions (Qiagen Plasmid Purification Handbook, 1999) but lysozyme treatment (1 mg/ml) was added to the protocol in order to degrade the peptidoglycan layer. Lysozyme was supplemented to buffer P1 and cells were incubated at 37°C for 30 minutes.

PCR was generally performed according to protocols described in WO03/040352.

### Characterization of amino terminal region of various forms of metallo-beta-lactamase

For determinations of the N-terminal amino acid sequence and mass spectrometry, purified metallo-beta-lactamase samples were subjected to reverse phase chromatography and the enzymes were fractioned in a linear gradient of 0.1 % of TFA and 0.075 % TFA- acetonitrile from 0% to 100 % for 60 minutes.

NH₂-terminal sequences of metallo-beta-lactamase forms were determined by automated Edman degradation with an Applied Biosystems model 494A Protein Sequenator.

Mass analyses of metallo-beta-lactamase forms were performed by a hybrid quadrupole-time-of-flight (Q-TOF) instrument. Metallo-beta-lactamase variants have been assumed to provide comparable ionization potentials which have been utilized in the assessments of their relative proportions in the samples.

The nucleotide sequences of the beta-lactamase genes were determined by the dideoxy-chain termination method with an automatic DNA sequencer.

### Example 2. The complete nucleotide sequence of Bacillus cereus 98ME1552 metallo-beta-lactamase gene

The determination of the complete gene coding metallo-betalactamase was sequentially performed by using PCR and Vectorette techniques. Part of the structural gene was amplified by PCR, with isolated chromosomal DNA of a clinical isolate *B. cereus* 98ME1552 as a template and with primers designed to hybridize to the SQKVEKTVI coding region (forward primer) and HTLDLL coding region (reverse primer) of the *Bacillus cereus* 569/H metallo-beta-lactamase gene. Both primers also carry *Hind* III restriction sites. The amplified DNA fragment (about 700 bp) was digested with *Hind* III and ligated to the *Hind* III site of a secretion vector pKTH141. Competent *Bacillus subtilis* IH6140 cells were transformed with the ligation mixture. A done harbouring a plasmid expressing metallo-beta-lactamase was verified by DNA sequencing. The plasmid was named pRSH314.

Competent *B.subtilis* RS303 cells prepared as described in WO03/04052 were transformed with pRSH314 resulting in strain *B. subtilis* RS314.

The complete DNA sequence of metallo-beta-lactamase gene was determined from PCR fragments obtained by employing Vectorette technique as follows: The chromosomal DNA of *Bacillus cereus* 98ME1552 was digested with *Hind* III restriction enzyme and ligated to *Hind* III treated Vector-ettell. The obtained Vectorette-library was screened in a PCR reaction by employing the MEBLSQ-F (5'-AGGAAATGTTGCGGATGC) or MEBLSQ-R (5'-CCTTCGTTAATTTGTTATCCC) initiating primers designed from the DNA sequence obtained from the 700 bp of pRSH314.

PCR screening of the Vectorette library with the MEBLSQ-F primer generated a fragment of about 1000 bp (MEBL1 fragment) and with the MEBLSQ-R primer a fragment of about 1100 bp (MEBL2 fragment). Both MEBL1 and MEBL2 fragments were purified from agarose gel after electrophoresis. The nucleotide sequences of both fragments were determined by DNA sequencing resulting in the complete nucleotide of *B. cereus* 98ME1552 metallo-beta-lactamase gene. The complete nucleotide and deduced amino acid sequence of the *B. cereus* 98ME1552 metallo-beta-lactamase gene is presented in Figure 1. The amino acid sequence is also set forth as SEQ ID NO: 1. The open reading frame encodes a 257 amino acid polypeptide, whose amino terminal sequence (30 amino acid residues) exhibits features typical of those of a bacterial signal peptide that targets protein secretion across the cytoplasmic membrane via a general secretory pathway. The predicted signal peptidase cleavage site is after alanine at position of -30 (see Figure 1.). The calculated molecular mass and the pl value of the mature protein of 227 amino acid residues is 24 877.3 Da and 6.0, respectively. The 98ME1552 metallobeta-lactamase sequence was used as a search template in a BLAST search in order to identify the protein. The BLAST search was performed using the SIB BLAST Network Service of Swiss Institute of Bioinformatics (http://www.expasy.org/tools/blast/). The search was done against a UniProtKB Knowledge based database using the NCBI BLASTP 2.2.13 program (Altschul *et al.,* 1997) with BLOSUM62 algorithm and gap penalties 11 for existence and 1 for extension. The BLAST search performed with the 98ME1552 metallo-beta-lactamase gave the highest similarity scored with other class B beta-lactamases of subgroup B1. As expected, the 98ME1552 beta-lactamase exhibited a high degree of identity (over 90 %) to other *B.cereus* beta-lactamases. According to structural alignment of various *B. cereus* metallo-beta-lactamase, the amino acid substitutions in *B. cereus* 98ME1552 lactamase are predicted to locate in regions not essential for the function of the enzyme.

### Example 3. Cloning and expression of the B.cereus 98ME1552 metallo-beta-lactamase gene in Bacillus subtilis

Based on the obtained DNA sequences from the Vectorette library, new primers, BLC1-F
(5'-CGCGAAGCTTCCGAACAAAAGCTAGAGCAAATAGTAATC),
   and BLC1-R
(5'-GCCGAAGCTTTTATTTTAATAAATCCAATGTATGTAAAAGTAATCCC)
were designed to generate a DNA insert encoding the complete metallo-beta-lactamase in PCR. The primers also carried *Hind* III-sites at their ends and purified chromosomal DNA of *B. cereus* 98ME1552 was used as a template. The amplified PCR fragment of about 0.7 kb was digested with *Hind* III and ligated to the *Hind* III site of a pKTH141 secretion vector. Competent *Bacillus subtilis* RS303 cells were transformed by the ligation mixture. One positive clone was designated RS315 and the harbouring expression construct was named pRSH315.

The pRSH315 expression construct was isolated and the insert region was sequenced. The nucleotide and deduced amino acid sequences of the cloned metallo-beta-lactamase gene were identical to those determined from the Vectorette library. The determined DNA sequence revealed in frame fusion between the nucleotide sequence encoding a 31 amino acid long signal sequence of *Bacillus amyloliquefaciens* alpha amylase, the *Hind* III cloning site and the complete *B.cereus* 98ME1552 metallo-beta-lactamase gene (see Figure 2.). Signal peptidase is predicted to cut the peptide bond between alanine (A) at position of -1 and glutamine (Q) at position of +1. The mature metallo-beta-lactamase possesses an NH₂-terminal extension of a NH₂-QAS-tripeptide that is derived from the *Hind* III cloning site in the expression construct. Hence, based on the deduced amino acid sequence the mature modified metallo-beta-lactamase is comprised of 230 amino acid residues.

### Example 4. Determination of the amino terminal amino acid sequence of metallo-beta-lactamase variants obtained from a Bacillus subtllis production system

For further characterization, the recombinant metallo-beta-lactamase was produced in cultivations performed in either shaking flask or fermentation by using a synthetic growth medium. The metallo-beta-lactamase was effectively secreted into the culture supernatant. The enzyme was purified from concentrated culture supernatant by ion exchange chromatography. Enzyme activity was observed in two separate fractions. Purified enzyme fractions were subjected to NH₂-terminal sequencing and mass spectrometry analysis. Analyses revealed that both fractions comprised enzyme forms with heterogeneous amino terminus. The various enzyme forms and their relative proportions are presented in Table 3. Related to the deduced amino acid sequence, all enzyme forms have deletions of various lengths in their NH₂-terminal regions. In general, the NH₂-QASEQKLE octapeptide seems to be deleted in all enzyme forms. Furthermore the smallest enzyme form in fraction 2 lacks an additional IVIKN-pentapeptide. The observed microheterogeneity in the NH₂-terminal region can be explained as post translational modifications caused by the action of various host cell proteases.

**Table 3. Deduced and determined amino terminal sequences and determined molecular mass of truncated metallo-beta-lactamase forms**

| **Enzyme fraction n:o** | **Deduced NH₂-terminal amino acid sequence** | **Determined NH₂**-**terminal sequence** | **Relative proportion of enzyme forms in fractions (%)** | **Determined Mass (kDa)** |
|---|---|---|---|---|
| | NH₂-QASEQKLEQIVIKNETGTI | | | |
| 1 | | NH₂-IVIKNETGTI | 100 | 24.122 |
| 2 | | NH₂-NETGTI | 60 | 23.668 |
| 2 | | NH₂-ETGTI | 40 | 23.554 |

### Example 5. Construction of Bacillus cereus 98ME1552 deleted metallo-beta-lactamase form and its amino terminal variants in a Bacillus subtilis production system

In order to try to reduce NH₂- terminal heterogeneity the DNA sequence encoding the variable region NH₂-EQKLEQIVIKN of the metallo-beta-lactamase gene was deleted by PCR. The complete gene of *B. cereus 98ME1552* metallo-beta-lactamase was used as a template in PCR with a forward primer hybridizing to a ETGTISISQ coding sequence and a reverse primer hybridizing to a GLLLHTLDLLK coding sequence followed by a translation stop codon TAA. Both primers carried *Hind* III sites.

The obtained PCR fragment of about 0.7 kb was cloned into the *Hind* III site of the pKTH141 secretion vector and the competent *B.subtilis* RS303 strain was transformed by the ligation mixture. The NH₂-terminal deletion was verified by DNA sequencing of the inserted metallo-beta-lactamase gene of the expression construct isolated from a positive clone. The transformant strain was named *Bacillus subtilis* RS317 and the expression construct was called pRSH317.

Truncated metallo-beta-lactamase was produced in a *Bacillus subtilis* production host and the enzyme was purified from the culture supernatant by employing ion change chromatography from which the active enzyme was eluted as a single fraction. The enzyme fraction was subjected to NH₂-terminal amino acid sequencing and molecular mass analysis. The observed results (in Table 4) showed that the truncated metallo-beta-lactamase also appeared as a protein with a heterogeneous amino terminus. The major enzyme variant possesses an amino- terminal sequence identical to that of the deduced amino acid sequence including the *Hind* III site coding the initiation QAS-tripeptide. A minor variant was found to have a deletion of a QA- dipeptide. Due to amino terminal blockage, no amino acid sequence was received from one variant. Amino terminal blockage is likely to be the result of cyclization of the glutamine residue to form a pyroglutamyl residue during the production process. To conclude, the amino terminal deletion did not fundamentally reduce amino terminal microheterogeneity.

**Table 4. Determined amino terminal sequences and molecular masses of truncated metallo-beta-lactamase forms**

| **Enzyme fraction n:o** | **Deduced NH₂**-**terminal amino acid sequence** | **Determined NH₂**-**terminal sequence** | **Relative proportion of enzyme forms in fractions (%)** | **Mass analysis (kDa)** |
|---|---|---|---|---|
| | NH₂-QASETGTISI | | | |
| 1. | | **a**.cyclic form of glutamine → no amino acid sequence | | **a**. 23.823 |
| | | **b**. NH₂-QASETGTISI | **b**. 90 | **b**. 23.840 |
| | | **c**. NH₂-SETGTISI | **c**. 10 | **c**. 23.641 |

### Example 6. Construction of Bacillus cereus 98ME1552 truncated metallo-beta-lactamase possessing a KT coding sequence insert

In order to avoid post translational modification a molecular truncation of metallo-beta-lactamase was designed to comprise the deletion of the DNA sequence coding the NH₂-EQKLEQIVIKN region together with insertion of a KT dipeptide coding sequence located directly downstream of the 3'- *Hind* III cloning site.

A modified metallo-beta-lactamase gene was created as in Example 5, except for the forward primer, which contained a KT coding DNA sequence. The PCR fragment was cut with *Hind* III and ligated to the *Hind* III site of the pKTH141 secretion vector and competent *B.subtilis* RS303 cells were transformed by the ligation mixture. The correct nucleotide sequence of the modified metallo-beta-lactamase gene in the expression construct was confirmed by DNA sequencing. One positive clone was named *Bacillus subtilis* RS318 and the expression construct was called pRSH318. The nucleotide and the deduced amino acid sequence of the *B. cereus* 98ME1552 beta-lactamase derived from pRSH318 is presented in Figure 3, and set forth as SEQ ID NO:2 and 3, respectively.

The truncated metallo-beta-lactamase was produced, purified, and analyzed as earlier described. The active truncated metallo enzyme was eluted from the column as a single peak. The single fraction contained metallo-beta-lactamase that possessed a homologous amino terminal glutamic acid residue (NH₂-E; see Table 5). Accordingly, the KT-dipeptide insertion conducts post translational modification resulting in a uniform amino terminal amino acid sequence. The truncated metallo-beta-lactamase was named the P2A protein.

**Table 5. Deduced and determined amino terminal sequences and the determined molecular mass of truncated metallo-beta-lactamase form**

| **Enzyme fraction n:o** | **Deduced NH₂**-**terminal amino acid sequence** | **Determined NH₂**-**terminal sequence** | **Relative proportion of enzyme forms in fractions (%)** | **Mass analysis (kDa)** |
|---|---|---|---|---|
| | NH₂-QASKTETGTISI | | | |
| 1. | | **a**.NH₂-ETGTISI | **a**. 100 | **a**.23554 |

### Example 7. Kinetic enzyme parameters of the P2A metallo-beta-lactamase

Diversity of the catalytic properties of the P2A enzyme was studied with various types of beta-lactams including the penicillin family with and without serine beta-lactamase inhibitors, second and third generation cephalosporins, and carbapenems (meropenem). The enzyme kinetic parameters k_{cat} and Kₘ were determined from initial rates by Hanes plot. The reactions were performed in 10 mM phosphate buffer, pH 7.0 at 30°C. The reaction cuvette (one mL) contained about 5 pmol of enzyme in all reactions except 1.7 pmol of enzyme in the meropenem assay. The hydrolysis of various beta-lactam substrates was spectrophometrically recorded at a wavelength specific for each substrate.

Values for different kinetic parameters (k_{cat}, Kₘ and k_{cat}/ Kₘ ) that represent mean values obtained from three independent measurements are reported in Table 6.

**Table 6. Kinetic parameter values for the P2A metallo-beta-lactamase**

| **Antibiotic** | **The P2A protein** | | |
|---|---|---|---|
| | Kₘ (microM) | k_{cat} (1/s) | k_{cat}/Kₘ (M⁻¹x s⁻¹) |
| Ampicillin | 942 | 1114 | 1.18x10⁻⁶ |
| Ampicillin-sulbactam (Unasyn) | 1104 | 1251 | 1.15x10⁻⁶ |
| Amoxycillin | 716 | 980 | 1.37x10⁻⁶ |
| Amoxycillin-clavulanic acid (Augmentin) | 717 | 990 | 1.38x10⁻⁶ |
| Piperacillin | 372 | 1049 | 2.82x10⁻⁶ |
| Piperacillin-tazobactam (Tazocin) | 412 | 1098 | 2.67x10⁻⁶ |
| Cefuroxime | 27 | 221 | 7.99x10⁻⁶ |
| Cefotaxime | 66 | 479 | 7.28x10⁻⁶ |
| Ceftriaxone | 68 | 95 | 1.40x10⁻⁶ |
| Meropenem | 410 | 480 | 1.17x10⁻⁶ |

### Example 8. Stability of the P2A protein in human ileal chyme

Resistance to intestinal proteases is one of the most important factors affecting the applicability of the P2A protein as a drug substance in targeted beta-lactamase therapy in the small intestine. The susceptibility of metallo-beta-lactamase to the action of small intestinal proteases was tested by adding various amounts of active enzyme in tubes consisting human ileal chyme. The hydrolysis of metallo-beta-lactamase was monitored by measuring beta-lactamase activity of ileal samples at various time points. Meropenem was employed as substrate in the activity assays.

The obtained results from four independent experiments and the mean values are expressed in Table 7. The P2A enzyme appears to be a stable protein, which was cleared in human ileal chyme with a half-life of 55 minutes (mean value). High variations of half-lives were observed between various experiments. However, the half-life of P2A in human small intestinal chyme has been evaluated to be adequate for successful application of P2A enzyme therapy for elimination of residual beta-lactam induced adverse reaction in the intestinal tracts.

**Table 7. Half-life (in vitro) of the P2A metallo-beta-lactamase in human ileal chyme.**

| Experiment n:o | Half-life (minutes) | Mean value (±SD) |
|---|---|---|
| 1 | 60 | 55±25 |
| 2 | 80 | 55±25 |
| 3 | 20 | 55±25 |
| 4 | 60 | 55±25 |

### References

Altschul S.F., Madden T.L., Schäffer A.A., Zhang J., Zhang Z., Miller W., Lipman D.J. 1997. Gapped BLAST and PSI-BLAST: a new generation of protein database search programs. Nucleic Acids Res. 25: 3389-3402
Ambler, R.P. 1980. The structure of beta-lactamases. Philos. Trans. R. Soc. London B 289:321-331.
Bush, K. 1998. Metallo-β-lactamases: a class apart. Clin. Infect. Dis. 32: 271-276.
Carfi A, Duee E, Galleni M, Frere JM, and Dideberg O. 1998a. 1.85 A resolution structure of the zinc (II) beta-lactamase from Bacillus cereus.Acta Crystallogr D Biol Crystallogr. 54:313-323.
Carfi A, Duee E, Paul-Soto R, Galleni M, Frere JM, and Dideberg O. 1998b. X-ray structure of the Znll beta-lactamase from Bacteroides fragilis in an orthorhombic crystal form. Acta Crystallogr D Biol Crystallogr. 54:45-57.
Carfl, A., Pares, S., Duee, E., Galleni, M., Duez, C., Frere, J.M., and Dideberg O. 1995. The 3-D structure of a zinc metallo-beta-lactamase from Bacillus cereus reveals a new type of protein fold. EMBO J. 14:4914-4921.
Concha, N.O., Janson, C.A., Rowling, P., Pearson, S., Cheever, C.A, Clarke, B.P., Lewis, C., Galleni, M., Free, J.M., Payne, D.J., Bateson, J.H., and Abdel-Meguld, S.S. 2000. Crystal structure of the IMP-1 metallo beta-lactamase from Pseudomonas aeruginosa and its complex with a mercaptocarboxylate inhibitor: binding determinants of a potent, broad-spectrum inhibitor. Biochemistry. 39:4288-4298.
Crawford P. A., et al., 2004. Over-expression, purification, and characterization of metallo-β-lactamase lmiS from Aeromonas veronii bv sobria. Protein Expression and Purification 36:272-279.
Galleni, M., Lamotte-Brasseur, J., Rossolini, G.M., Spencer, J., Dideberg, O., and Frere, J.M. 2001. Metalio-beta-lactamases Standard numbering scheme for class B beta-lactamases. Antimicrob. Agents Chemother. 45:660-663
Garau, G., Garcia-Saez, I., Bebrone, C., Anne, C., Mercuri, P., Galleni, M., Frere, J.M., and Dideberg, O. 2004. Update of the standard numbering scheme for class B beta-lactamases. Antimicrob. Agents Chemother. 48:2347-2349.
Garau, G., Bebrone, C., Anne, C., Galleni, M., Frere, J.M., Dideberg, O. 2005. A metallo-beta-lactamase enzyme in action: crystal structures of the monozinc carbapenemase CphA and its complex with biapenem. J Mol Biol.345:785-795.
Harmoinen, J., Mentula, S., Heikkilä, M., van der Rest M., Rajala-Schultz, P.J., Donskey, C.J., Frias, R., Koski, P., Wickstrand, N., Jousimies-Somer, H., Westermarck, E., Llndevall, K. 2004. Oral Targeted Recombinant Beta-Lactamase Prevents Ampicillin-Induced Selective Pressure on the Gut Microbiota: A Novel Approach to Reduce Antimicrobial Resistance Antimicrob. Agents and Chemotherapy. 48: 75-79.
Marmur, J. 1961. A procedure for the isolation of deoxyribonucleic acid from micro-organisms. J. Mol. Biol. 3: 208-218.
Mentula, S., Harmoinen, J., Koskl, P., Westermarck, E., Huovinen, P., and Könönen, E. 2004. Inhibition of ampicillin-Induced emergence of resistance in intestinal coliforms by targeted recombinant beta-lactamase. Int.J.Antimicrob.Agents. 24:555-561.
Sambrook, J., and Russell, D.W. 2001. Molecular cloning. a Laboratory Manual. Cold Spring Harbour Laboratory Press Cold Spring Harbour, New York.
Simm A. M., 2002. Characterization of Monomeric L1 Metallo-β-lactamase and the Role of the N-terminal Extension in Negative Cooperativity and Antibiotic Hydrolysis. The Journal of Biological Chemistry 277(27):24744-24752.
Stiefel, U., Pultz, N.J., Harmoinen, J., Koski, P., Lindevall, K., Helfand, M.S., Donskey, C.J. 2003. Oral β-lactamase administration preserves colonization resistance of piperacillin-treated mice. J Infect Dis. 10: 1605-1609.
Stiefel, U., Harmoinen, J., Koski, P., Kaariainen, S., Wickstrand, N., Lindevall, K., Pultz, N.J., Bonomo, R.A., Helfand, M.S., and Donskey, C.J. 2005. Orally administered recombinant metallo-beta-lactamase preserves colonization resistance of piperacillin-tazobactam-treated mice. Antimicrob. Agents Chemother.49: 5190-5191.
Walsh, T.R., Toleman, M.A., Poirel, L, and Nordmann, P. 2005. Metallo-beta-lactamases: the quiet before the storm? Clin Microbiol Rev. 18:306-325
Walther-Rasmussen J. et al., 1999. Terminal trunctions in Amp C β-lactamase from a clinical isolate of Pseudomonas aeruginosa. Eur. J. Biochem 263:478-485.

## Claims

1. A modified metallo-beta-lactamase protein having the general formula:
NH₂-K-T-E-ΔBL-COOH
wherein
K is lysine
T is threonine
E is glutamic acid, and
ΔBL is a metallo-beta-lactamase protein that has been truncated at the amino terminal end so as to leave four beta-strands before the first alpha-helix of the predicted secondary structure of said protein, and the sequence E - ΔBL has at least 70 % identity to the amino acid sequence of sequential amino acid residues 6-221 of SEQ ID NO:3.

2. An isolated nucleotide molecule comprising a nucleotide sequence encoding the metallo-beta-lactamase protein of claim 1.

3. The nucleotide molecule of claim 2, comprising sequential nucleotides 94-756 of SEQ ID NO:2.

4. An expression vector containing the nucleotide molecule of claim 2.

5. A host cell capable of expressing the metallo-beta-lactamase protein encoded by the nucleotide molecule of claim 2.

6. The host cell of claim 5, which secretes said metallo-beta-lactamase protein.

7. The host cell of claim 5 or 6, which is *Bacillus* spp, especially *B*. *licheniformis* or *B. subtilis.*

8. A modified metallo-beta-lactamase protein having the general formula
NH₂- E - ΔBL - COOH
wherein
E is glutamic acid, and
ΔBL is a metallo-beta-lactamase protein that has been truncated at the amino terminal end so as to leave four beta-strands before the first alpha-helix of the predicted secondary structure of said protein, and the sequence E - ΔBL has at least 70 % identity to the amino acid sequence of sequential amino acid residues 6-221 of SEQ ID NO:3.

9. The metallo-beta-lactamase protein of claim 8, which belongs to subgroup B1 of metallo-beta-lactamases.

10. The metallo-beta-lactamase protein of claim 9, wherein the truncated beta-lactamase originates from *Bacillus* spp., and especially from *B*. *cereus*.

11. The metallo-beta-lactamase of claim 10, wherein E - ΔBL is derived by deleting the first eleven amino terminal amino acids of a mature metallo-beta-lactamase.

12. The metallo-beta-lactamase protein of claim 9, wherein the sequence E - ΔBL has at least 80 % identity to the amino acid sequence of sequential amino acid residues 6-221 of SEQ ID NO:3.

13. The metallo-beta-lactamase protein of claim 9, wherein the sequence E - ΔBL is a truncated form of a metallo-beta-lactamase having the sequence set forth as SEQ ID NO:1, or a beta-lactamase active fragment thereof.

14. The metallo-beta-lactamase of claim 9, wherein E - ΔBL is derived by truncation of a metallo-beta-lactamase between the amino acids KN and E.

15. A method of preparing a modified metallo-beta-lactamase protein, said method comprising culturing a host cell of claim 5 under conditions enabling the expression of a metallo-beta-lactamase having the general formula:
NH₂-K-T-E-ΔBL-COOH,
wherein
K is lysine
T is threonine
E is glutamic acid, and
ΔBL is a metallo-beta-lactamase protein that has been truncated at the amino terminal end so as to leave four beta-strands before the first alpha-helix of the predicted secondary structure of said protein, and the sequence E - ΔBL has at least 70 % identity to the amino acid sequence of sequential amino acid residues 6-221 of SEQ ID NO:3
and conducting post translational modification resulting in a modified metallo-beta-lactamase having the general formula:
NH₂-E-ΔBL-COOH,
wherein Band ΔBL are as defined above, and
optionally isolating and purifying the post translationally modified protein obtained.

16. The method of claim 15, wherein the metallo-beta-lactamase protein is expressed in a form comprising a signal sequence, whereby the protein is secreted from the host cell.

17. The method of claim 15 or 16, wherein the protein is produced in *Bacillus* spp. especially in *B. licheniformis* or *B. subtilis.*

18. A pharmaceutical composition comprising the modified metallo-beta-lactamase protein of claim 8.

19. The modified metallo-beta-lactamase of claim 8 for use as a medicament.

20. Use of the modified metallo-beta-lactamase of claim 8 for the manufacture of a medicament for elimination of beta-lactam antibiotic induced adverse effects in the intestinal tract.

21. The use of claim 20, wherein the beta-lactam antibiotic is selected from the group consisting of cephalosporins, carbapenems and penicillins, which antibiotic is to be eliminated in the presence or absence of an inhibitor against serine beta-lactamases.

## Patentansprüche

1. Modifiziertes Metallo-β-Lactamase-Protein der allgemeinen Formel:
NH₂₋K-T-E-ΔBL-COOH
worin
K Lysin bedeutet,
T Threonin bedeutet,
E Glutaminsäure bedeutet und
ΔBL ein Metallo-β-Lactamase-Protein bedeutet, das am aminoterminalen Ende so verkürzt wurde, dass 4 β-Stränge vor der ersten α-Helix der vorhergesagten sekundären Struktur des Proteins übrig bleiben, und die Sequenz E-ΔBL zumindest 70 % Identität mit der Aminosäuresequenz der sequenziellen Aminosäurereste 6-221 von SEQ ID Nr. 3 aufweist.

2. Isoliertes Nucleotidmolekül, umfassend eine Nucleotidsequenz, welche das Metallo-β-Lactamase-Protein gemäß Anspruch 1 codiert.

3. Nucleotidmolekül nach Anspruch 2, umfassend die sequenziellen Nucleotide 94-756 von SEQ ID Nr. 2.

4. Expressionsvektor, enthaltende das Nukleotidmolekül nach Anspruch 2.

5. Wirtszelle, die zur Expression des durch das Nukleotidmolekül nach Anspruch 2 codierten Metallo-β-Lactamase-Proteins befähigt ist.

6. Wirtszelle nach Anspruch 5, die das Metallo-β-Lactamase-Protein sezerniert.

7. Wirtszelle nach Anspruch 5 oder 6, die *Bacillus* spp., insbesondere *B. licheniformis* oder *B*. *subtilis* darstellt.

8. Modifiziertes Metallo-β-Lactamase-Protein der allgemeinen Formel:
NH₂-E-ΔBL-COOH
worin
E Glutaminsäure bedeutet und
ΔBL ein Metallo-β-Lactamase-Protein bedeutet, das am terminalen Aminoende so gekürzt wurde, dass 4 β-Stränge vor der ersten α-Helix der vorhergesagten sekundären Struktur des Proteins übrig bleiben, und die Sequenz E-ΔBL zumindest 70 % Identität mit der Aminosäuresequenz der sequenziellen Aminosäurereste 6-221 von SEQ ID Nr. 3 aufweist.

9. Metallo-β-Lactamase-Protein nach Anspruch 8, das zur Untergruppe B1 der Metallo-β-Lactamasen gehört.

10. Metallo-β-Lactamase-Protein nach Anspruch 9, wobei die gekürzte β-Lactamase aus *Bacillus* spp., und insbesondere aus *B. cereus* stammt.

11. Metallo-β-Lactamase-Protein nach Anspruch 10, wobei E - ΔBL durch Deletion der ersten 11 endständigen Aminosäuren der reifen Metallo-β-Lactamase abgeleitet wurde.

12. Metallo-β-Lactamase-Protein nach Anspruch 9, wobei die Sequenz E - ΔBL zumindest 80 % Identität mit der Aminonsäuresequenz der sequenziellen Aminosäurereste 6-221 von SEQ ID Nr. 3 aufweist.

13. Metallo-β-Lactamase-Protein nach Anspruch 9, wobei die Sequenz E - ΔBL eine gekürzte Form einer Metallo-β-Lactamase mit der als SEQ ID NO. 1 angegebenen Sequenz oder eine β-Lactamase-aktives Fragment davon ist.

14. Metallo-β-Lactamase-Protein nach Anspruch 9, wobei E - ΔBL durch Kürzung einer Metallo-β-Lactamase zwischen den Aminosäuren KN und E erhalten wurde.

15. Verfahren zur Herstellung eines modifizierten Metallo-β-Lactamase-Proteins, umfassend die Züchtung einer Wirtszelle nach Anspruch 5 unter Bedingungen, welche die Expression einer Metallo-β-Lactamase der allgemeinen Formel:
NH₂-K-T-E-ΔBL-COOH
worin
K Lysin bedeutet,
T Threonin bedeutet,
E Glutaminsäure bedeutet und
ΔBL ein Metallo-β-Lactamase-Protein bedeutet, das am aminoterminalen Ende so verkürzt wurde, dass 4 β-Stränge vor der ersten α-Helix der vorhergesagten sekundären Struktur des Proteins übrig bleiben, und die Sequenz E-ΔBL zumindest 70 % Identität mit der Aminosäuresequenz der sequenziellen Aminosäurereste 6-221 von SEQ ID Nr. 3 ermöglicht,
und die Durchführung einer posttranslationalen Modifikation zu einer modifizierten Metallo-β-Lactamase der allgemeinen Formel:
NH₂-E-ΔBL-COOH
worin E und ΔBL wie oben definiert sind, und
gegebenenfalls die Isolierung und Reinigung des erhaltenen posttranslational modifizierten Proteins.

16. Verfahren nach Anspruch 15, worin das Metallo-β-Lactamase-Protein in einer Form exprimiert wird, die eine Signalsequenz umfasst, wodurch das Protein aus der Wirtszelle sezerniert wird.

17. Verfahren nach Anspruch 15 oder 16, wobei das Protein in *Bacillus* spp., insbesondere in *B. licheniformis* oder *B. subtilis* erzeugt wird.

18. Pharmazeutische Zusammensetzung, enthaltend das modifizierte Metallo-β-Lactamase-Protein nach Anspruch 8.

19. Modifizierte Metallo-β-Lactamase nach Anspruch 8 zur Verwendung als Medikament.

20. Verwendung der modifizierten Metallo-β-Lactamase nach Anspruch 8 zur Herstellung eines Medikaments zur Beseitigung von durch β-Lactam-Antibiotikum hervorgerufenen ungünstigen Wirkungen im Darmtrakt.

21. Verwendung nach Anspruch 20, wobei das β-Lactam-Antibiotikum ausgewählt ist aus der Gruppe, bestehend aus Cephalosporinen, Carbapenemen und Penizillinen, wobei das jeweilige Antibiotikum in Anwesenheit oder Abwesenheit eines Inhibitors gegen Serin-β-Lactamasen zu entfernen ist.

## Revendications

1. Protéine métallo-bèta-lactamase modifiée de formule générale :
NH₂-K-T-E-ΔBL-COOH
dans laquelle
K est lysine
T est thréonine
E est acide glutamique, et
ΔBL est une protéine métallo-bèta-lactamase, qui a été tronquée à l'extrémité terminale amino de manière à laisser quatre brins bèta avant la première hélice alpha de la structure secondaire prédite de cette protéine, et la séquence E-ΔBL a au moins une identité à 70% avec la séquence d'acide aminé des résidus d'acide aminé en séquence 6 à 221 de l'identification de séquence N° 3.

2. Molécule de nucléotide isolée comprenant une séquence de nucléotides codant la protéine métallo-bèta-lactamase de la revendication 1.

3. Molécule de nucléotide suivant la revendication 2, comprenant des nucléotides en séquence 94 à 756 de l'identification de séquence N° 2.

4. Vecteur d'expression contenant la molécule de nucléotide de la revendication 2.

5. Cellule hôte apte à exprimer la protéine métallo-bèta-lactamase codée par la molécule de nucléotide de la revendication 2.

6. Cellule hôte suivant la revendication 5, qui sécrète la protéine métallo-bèta-lactamase.

7. Cellule hôte suivant la revendication 5 ou 6, qui est *Bacillus* spp, en particulier *B licheniformis* ou *B subtilis.*

8. Protéine métallo-bèta-lactamase modifiée ayant la formule générale :
NH₂-E-ΔBL-COOH
dans laquelle
E est acide glutamique, et
ΔBL est une protéine métallo-bèta-lactamase, qui a été tronquée à l'extrémité terminale amino de manière à laisser quatre brins bèta avant la première hélice alpha de la structure secondaire prédite de cette protéine, et la séquence E-ΔBL a au moins une identité à 70% avec la séquence d'acide aminé des résidus d'acide aminé en séquence 6 à 221 de l'identification de séquence N° 3.

9. Protéine métallo-bèta-lactamase suivant la revendication 8, qui appartient à des sous-groupes B1 de métallo-béta-lactamases.

10. Protéine métallo-bèta-lactamase suivant la revendication 9, dans laquelle la bèta-lactamase tronquée provient de *Bacillus* spp et en particulier de *B cereus.*

11. Protéine métallo-bèta-lactamase suivant la revendication 9, dans laquelle E-ΔBL est dérivé par suppression des onze premiers acides aminés sur la terminaison amino d'une métallo-bèta-lactamase mature.

12. Protéine métallo-bèta-lactamase suivant la revendication 9, dans laquelle la séquence **E-ΔBL** a une identité à au moins 80% avec la séquence d'acide aminé des restes d'acide aminé en séquence 6 à 221 de l'identification de séquence N° 3.

13. Protéine métallo-bèta-lactamase suivant la revendication 9, dans laquelle la séquence **E-**Δ**BL** est une forme tronquée d'une métallo-bèta-lactamase ayant la séquence donnée comme identification N° 1 ou en est un fragment actif de bèta-lactamase.

14. Protéine métallo-bèta-lactamase suivant la revendication 9, dans laquelle **E-**Δ**BL** provient de la troncature d'une métallo-bèta-lactamase entre les acides aminés **KN** et **E.**

15. Procédé de préparation d'une protéine métallo-bèta-lactamase modifiée, le procédé comprenant cultiver d'une cellule hôte suivant la revendication 5 dans des conditions permettant l'expression d'une métallo-bèta-lactamase de formule générale :
**NH₂-K-T-E-ΔBL-COOH**
K est lysine
**T est** thréonine
**E** est acide glutamique, et
**ΔBL** est une protéine métallo-bèta-lactamase, qui a été tronquée à l'extrémité terminale amino de manière à laisser quatre brins bèta avant la première hélice alpha de la structure secondaire prédite de cette protéine, et la séquence **E-**Δ**BL** a au moins une identité à 70% avec la séquence d'acide aminé des résidus d'acide aminé en séquence 6 à 221 de l'identification de séquence N° 3,
et effectuer une modification de post-traduction donnant une métallo-bèta-lactamase modifiée de formule générale :
**NH₂-E-ΔBL-COOH**
dans laquelle E et ΔBL sont tels que définis ci-dessus, et
facultativement, isoler et purifier la protéine modifiée en ayant subi une post-traduction, qui a été obtenue.

16. Procédé suivant la revendication 15, dans lequel on exprime la protéine métallo-bèta-lactamase sous une forme comprenant une séquence signal, la protéine étant sécrétée par la cellule hôte.

17. Procédé suivant la revendication 15 ou 16, dans lequel on produit la protéine dans *Bacillus* spp, en particulier dans *B licheniformis* ou *B subtilis.*

18. Composition pharmaceutique comprenant la métallo-bèta-lactamase protéine modifiée suivant la revendication 8.

19. Métallo-bèta-lactamase modifiée suivant la revendication 8, à utiliser comme médicament.

20. Utilisation de la métallo-bèta-lactamase modifiée suivant la revendication 8, pour la fabrication d'un médicament d'élimination des effets secondaires induits par un bèta-lactame antibiotique dans le tractus intestinal.

21. Utilisation suivant la revendication 20, dans laquelle le bèta-lactame antibiotique est choisi dans le groupe consistant en des céphalosporines, des carbapénèmes et des pénicillines antibiotiques à éliminer en la présence ou en l'absence d'un inhibiteur à l'encontre de sérine béta-lactamases.
